(19) Europäisches Patentamt
European Patent Office
Office européen des brevets

(11) Publication number: 0 390 612 A1

(12) # EUROPEAN PATENT APPLICATION

(21) Application number: 90303499.9

(51) Int. Cl.[5]: C12P 21/08, C07K 3/00

(22) Date of filing: 02.04.90

(30) Priority: 31.03.89 US 332264

(43) Date of publication of application:
03.10.90 Bulletin 90/40

(84) Designated Contracting States:
CH DE FR GB IT LI SE

(71) Applicant: SYNBIOTICS CORPORATION
11011 Via Frontera
San Diego California 92127(US)

(72) Inventor: Maggio, Edward T.
18775 Bernardo Trails Drive
San Diego California 92128(US)

(74) Representative: Sheard, Andrew Gregory et al
Kilburn & Strode 30, John Street
London WC1N 2DD(GB)

(54) **Drug production using non-cross reactive antibodies.**

(57) A method for producing low molecular weight compounds, which are capable of binding to a naturally occurring receptor and which can be used as drugs and which mimic the three dimensional surface structure of antibodies to drug molecules, are produced by first generating a non-cross reactive antibody to a drug and then producing a low molecular weight structural mimic of the antibody.

EP 0 390 612 A1

Xerox Copy Centre

## DRUG PRODUCTION USING NON-CROSS REACTIVE ANTIBODIES

The invention relates generally to methods for producing ligands which bind to receptors. More particularly, the invention relates to the use of non-cross-reactive antibodies in the production of the ligands.

Monoclonal antibodies are immunoglobulins produced by hybridoma cells. A monoclonal antibody reacts with a single antigenic determinant and provides greater specificity than conventional, serum-derived polyclonal antibodies. Furthermore, screening a large number of monoclonal antibodies makes if possible to select an individual antibody with desired specificity, avidity and isotype. Hybridoma cell lines provide a constant, inexpensive source of chemically identical antibodies and preparations of such antibodies can be easily standardised. Methods for producing monoclonal antibodies are well known to those of ordinary skill in the art, eg, US-A-4198265 and US-A-4792528; Köhler and Milstein (1975) Nature 256:495 and Schook, ed. Monoclonal Antibody Production Techniques and Applications, published by Marcel Decker (1987).

Antigen recognition by a monoclonal antibody is attributable to a specific combining site in the N-terminal region of the immunoglobulin (Ig) molecule. Ig molecules are thought to react with antigens via the same types of short range forces characteristic of all protein-protein interactions. Antigen-antibody interactions are highly specific because of the complementary three-dimensional shapes of the antibody's combining site and of the corresponding antigenic determinant or epitope. Such complementary shapes permit the molecules to approach each other closely and to interact over a substantial surface area. The specificity of antibody-antigen interactions is evidenced by the fact that even slight changes in the configuration of the antigenic determinant result in marked decreases in the binding constant of the antigen to the antibody.

The biological and pharmacological properties of drugs, hormones, and other biological modifiers are determined to a large degree by the way these ligands bind specific receptors in the body. As a growing part of drug development, naked receptors or cells expressing specific receptor populations are being adapted as bioassays to screen chemical libraries for pharmaceutical leads. Computer-generated models, based on physical data, are also being used to predict ligand-receptor binding.

These forms of receptor technology (RT) are useful, but limited: only a fraction of important biological receptors are known and available as screening reagents. Furthermore, bioassays using natural receptors are often labile, subject to artefacts, and may contain only low concentrations of a desired receptor. Computer simulations, too, are hampered by the lack of structural data for many receptors.

Now, however, immunologists are beginning to apply their expertise to the problem. The antigen binding sites of antibodies can be designed to mimic the structure of a variety of biological receptors. The resulting receptor "models" can then be used as chemical screens in the discovery and tailoring of new drugs and other bioactive compounds. In fact, drug companies are beginning to utilise this approach to enhance and diversity standard bioassays and to extend advances in RT.

A drug's effects often result from complex interactions that may involve an initial ligand, secondary metabolic products, and a heterogeneous population of receptors operating under variable physiological conditions. Still, for many bioactive compounds, the ability to bind specifically a given population of receptors correlates well with biological activity.

The ability to use ligands as antigens from which to derive antibodies that mimic receptors is extremely valuable. Not all anti-ligand antibodies, however, mimic the ligand's receptor(s). Where the ligand contains multiple epitopes, and where only some of these correspond to the ligand's receptor-binding region, only subpopulations of antibody would be expected to work.

Antibodies may be generated to virtually any drug or other low molecular weight substances referred to as haptenic substances, by attachment of these low molecular weight substances to a carrier molecule such as a protein or other polymeric material. The site of attachment and the length of the linker arm between the haptenic substance and the macromolecular carrier molecule determine how the hapten is presented to the immune system. This in turn determines the nature of the immune response. If, for example, a drug has a number of epitopes to which antibodies can be generated, and this is typically the case, attachment of the drug to a macromolecule by epitope A will result in the generation of antibodies against epitopes B, C, D, etc, and a lack of response to epitope A. Likewise if the drug is attached by epitope B this will result in an immunogen capable of generating antibodies against epitopes A, C, D, etc. This knowledge has allowed scientists to generate specific immunogens which permit the generation of antibodies which are non-cross-reactive to other substances. For example, if a drug molecule and related molecules have a common structural feature, attachment of the drug away from this common structural feature will generate many crossreactive antibodies in an immunised animal, ie, the antibodies generated will recognise this common feature in a multitude of different structures. Conversely, if this common feature is used as an attachment

site, antibodies are not generated against this portion of the molecule and the antibodies are more likely to be specific for the hapten of interest. Generally speaking, attachment of haptens through common features and as distal as possible from unique or non-common features result in the greatest specificity in generating an antibody response.

PCT application WO-A-8606487 published 6th November 1986 discloses a method for producing a low molecular weight structural mimic or mimotope which has a surface region with the equivalent molecular topology to the binding surface of the ligand molecule of which it is the mimic. For example, in the context of immunological receptors, the mimotope mimics the epitope of the antigen. The mimotope which is produced by this method may not have any obvious or direct relationship to the natural ligand molecule, but will share with it the ability to react with the receptor. Such a mimotope can be used to replace the natural ligand in the treatment or prevent of a particular condition or disease or it may be used to mediate a particular biological effect.

PCT application WO-A-8800472 published 28th January 1988 discloses immunogens and methods of making immunogens capable of inducing active immunity against a given agent. The method comprises generating antibodies against the target cell receptor to which the agent binds. The antibodies which mimic the agent's receptor - specific binding site, can be used as immunogens to elicit antibodies in a host which resemble the receptor's binding site and are capable of binding with the agent's ligand thereby preventing the binding of that ligand to the target cell receptor.

Venter et al, eds Monoclonal and Anti-idiotypic Antibodies as Probes for Receptor Structure and Function, Alan R. Liss, Inc., New York (1984) disclose a collection of studies on monoclonal and anti-idiotypic antibodies for receptor characterisation.

Linthicum et al, in Bio Essays, Vol 3, No. 5, pp 213-217, 1985: "Using Molecular Mimicry to Produce Anti-receptor Antibodies" disclose that antibodies directed against pharmacologically active ligands have a three-dimensional binding site which is somewhat analogous to the natural receptor and that anti-idiotypic antibodies produced against these anti-ligand antibodies can have a three-dimensional shape which mimics the original ligand. The anti-idiotypic antibodies generated in this fashion are able to bind to cell surface receptors.

US-A-4262089 to Singh, et al, discloses theophylline derivatives useful in competitive protein binding assays. Theophylline is substituted at the 3 position and conjugated to antigens for production of antibodies which specifically recognise theophylline as distinct from structurally similar analogues such as caffeine.

It has now been determined that non-cross reactive antibodies may be of enormous commercial value in the rational design and production of drugs or hormone analogues or in the production of low molecular weight replacements of naturally occurring polypeptide drugs such as insulin, growth hormone, erythropoietin, interleukin, interferon or the like.

The invention therefore relates to a method of producing a low molecular weight ligand which is capable of binding to a naturally occurring receptor comprising producing non-crossreactive antibodies to a haptenic substance and producing the ligand as a low molecular weight structural mimic of the antibody.

The invention further relates to a method of producing a low molecule weight ligand capable of specific binding to a naturally occurring receptor comprising the sequential steps of (1) attaching a haptenic substance to a macromolecular carrier at a point on the haptenic substance having a common structural feature to related haptenic substances, (2) introducing the resulting carrier-haptenic combination into a live animal to produce non-crossreactive antibodies to the haptenic substance, (3) harvesting said antibodies and (4) producing the ligand as a low molecular weight structural mimic of said antibody which selectively binds to said receptor.

More particularly, the invention relates to a method of producing a low molecular weight ligand capable of bronchodilation comprising the sequential steps of (1) attaching theophylline to a macromolecular carrier at a point on the theophylline molecule having the common structural features of methyl xanthines, (2) introducing the resulting carrier-theophylline combination into a live animal to produce non-crossreactive antibodies, and (4) producing the ligand as a low molecular weight structural mimic of said antibody having bronchodilation activity.

The invention may broadly be seen as the use of non-crossreactive antibodies in the production of a compound capable of binding to a naturally occurring receptor.

As used throughout this specification, the terms listed below have the following meanings:

Receptor:

A molecule or molecular complex which will combine specifically with its particular ligand molecule. It is

those receptors which on binding with their particular ligand(s) mediate a biological function that are of most interest. Examples of receptors include, but are not restricted to, the common class of receptors associated with the surface membrane of cells and include, for instance, the immunologically important receptors of B-cells, T-cells, macrophages and the like. Another example is receptors for acetyl choline on nerve cells which cause a nerve pulse to be transmitted down the length of the neuron when the receptor molecule reacts with its ligand, acetyl choline.

Epitope:

The specific surface of an antigen molecule which is delineated by the area of interaction with the sub-class of receptors known as antibodies.

Catamer:

A polymer molecule which is a precisely defined linear sequence formed by the condensation of small molecules. Note that this term includes molecules in which different types of condensation reactions are used to join the small molecules. A number prefixed to the word "catamer" implies that the catamer is formed by the condensation of the indicated number of small molecules, for example, 8-catamer means that the catamer is made up from eight small molecules. Examples of catamers include any given peptide and any given oligosaccharide.

Monomer:

A member of the set of small molecules which can be condensed together to form a catamer. The set of monomers includes but is not restricted to, for example, the set of common L-amino acids, the set of D-amino acids, the set of synthetic amino acids, the set of nucleotides, and the set of pentoses and hexoses.

Peptide:

A catamer in which the small molecules are alpha-amino acids and which are joined together through a peptide bond. In the context of this specification it should be appreciated that the amino acids may be the L-optical isomer or the D-optical isomer.

Mimotope:

A catamer which in at least one of its conformations has a surface region with the equivalent molecular topology to the binding surface of the ligand molecule of which it is the mimic. In the context of immunological receptors, the mimotope mimics the epitope of the antigen.

Complementary:

Refers to the matching together of the reacting surfaces of a ligand molecule and its receptor. Thus, the receptor and its ligand can be described as complementary, and furthermore, the contact surface characteristics are complementary to each other.

Paratope:

The combining site of an antibody which is complementary to a particular epitope.

Ligand Molecule:

Is the molecule which binds to a particular receptor and when bound to it mediates the biological function associated with that particular receptor.

Low molecular weight:

Those skilled in the art will understand the meaning of the term as it is used in this specification. Preferred low molecular weights include those below 10kDa, below 5kDa or even below 1kDa.

Concept of Receptor

- Some drugs exhibit biological activity in minute concentrations. For this reason they are described as structurally specific. The effect produced by them is attributed to interaction with a specific cellular component known as a receptor. As a result of this interaction the drug forms a complex with the receptor.

It is assumed that structurally specific drugs present a high degree of molecular complementarily toward the site at which they act. The interaction of a drug of this type with its receptor would therefore be similar to the interaction of a substrate with the active site of an enzyme or of a hapten with an antibody.

Nature of Receptors

- Receptors explain the nature of the interaction of drugs with the living organisms to produce a specific biological effect.

Experimental evidence indicates that receptors are integral parts of certain macromolecules of living organisms. In most instances, they are segments of proteins. Often, they are the active sites themselves and sometimes the allosteric sites of enzymes. In some cases, receptors are parts of nonenzyme proteins. Receptors of many chemotherapeutic agents are nucleic acids (DNA or RNA), which can react chemically with these drugs through covalent bond or lodge them between their base pairs and the phosphate groups, by complexing with them by means of relatively weak interactions. Receptors can also be parts of lipoprotein complexes, principally of cellular membranes. The following table lists common drugs and their receptors or sites of action:

**Receptors or Sites of Action of Some Drugs**

| Drug | Receptor or Site of Action |
|---|---|
| Acetazolamide | carbonate dehydratase |
| acridines | DNA |
| actinomycins | DNA |
| adrenomimetics | adenylate cyclase |
| alkylating agents | DNA |
| allopurinol | xanthine oxidase |
| aminosalicylic acid | dihydropteroate synthase |
| amodiaquine | DNA |
| amphotericin B | cell membrane |
| anticholinesterases | acetylcholinesterase |
| antifolates | dihydrofolate reductase |
| aspirin | prostaglandin synthase |
| captopril | dipeptidyl carboxypeptidase I |
| carbidopa | aromatic L-amino-acid decarboxylase |
| carcinogens | DNA |
| cardiac glycosides | $Na^+/K^+$-transporting ATPase |
| chloramphenicol | peptidyltransferase (?) |

| Drug | Receptor or Site of Action |
|---|---|
| chlorguanide | dihydrofolate reductase |
| chloroquine | DNA |
| clavulanic acid | ß-lactamase |
| dapsone | dihydropteroate synthase |
| daunorubicin | DNA |
| disulfiram | dopamine ß-monooxygenase |
| erythromycin | ribosomes |
| fluorouracil | thymidylate synthase |
| fusidic acid | ribosomes |
| homidium bromide | DNA |
| hycanthone | DNA |
| idoxuridine | DNA |
| kanamycin | ribosomes |
| lincomycin | ribosomes |
| local anaesthetics | cell membrane |
| lucanthone | DNA |
| MAO inhibitors | amine oxidase |
| mepacrine | DNA |
| methotrexate | dihydrofolate reductase |
| methylxanthines | phosphodiesterase |
| mitomycin | DNA |
| nalidixic acid | DNA topoisomerase (ATP-hydrolysing) |
| neostigmine | acetylcholinesterase |
| organophosphorus insecticides | acetylcholinesterase |
| piperazine | succinate dehydrogenase |
| plicamycin | DNA |
| porphyromycin | DNA |
| pyrimethamine | dihydrofolate reductase |
| quinine | DNA |
| quinoline antimalarials | DNA |

| Drug | Receptor or Site of Action |
|---|---|
| rifampin | DNA-directed RNA polymerase |
| rifamycins | DNA-directed RNA polymerase |
| salicylates | prostaglandin synthase |
| schistosomicidal antimonials | 6-phosphofructokinase |
| streptomycin | ribosomes |
| sulbactam | ß-lactamase |
| sulphonamide diuretics | carbonate dehydratase |
| sulphonamides | dihydropteroate synthase |
| sulphones | dihydropteroate synthase |
| tetracyclines | ribosomes |
| thiabendazole | succinate dehydrogenase |
| trimethoprim | dihydrofolate reductase |

Complexation of a drug with special chemical groups on the receptor results in a sequence of chemical or conformational changes that either cause or inhibit biological reactions. Nowadays it is acknowledged that such changes in biopolymers actually occur as an effect of the action of small molecules. A drug's ability to adapt itself to a receptor depends on the structural, configurational, and conformational characteristics of both drug and receptor.

Isolation of Drug Receptors

- Several attempts have been made to isolate drug receptors, but success as yet has been equivocal. Difficulties in separating the receptor from tissue proteins are great, because during the process of extraction the forces that unite both entities - drug and receptor - are broken. Concomitantly, owing to changes in the structural shape of the macromolecule of which the receptor is an integral part, the functionality of this macromolecule can be destroyed. Furthermore, in the isolation process the receptor undergoes changes in its natural spatial arrangement and charge distribution, and both of these factors are essential to its interaction with the drug.

However, even the isolation of the receptors and the unequivocal identification of the functional groups involved in the interaction with drugs should not be a reason for excessive optimism because, just as the isolation of enzymes has not clarified how most of them function, the isolation of receptors may not elucidate thoroughly the mechanism of action of drugs with which they complex.

Structure

- Despite the little that is known about the subject, it is generally accepted that a receptor is an elastic three-dimensional entity, consisting in most cases of protein-constituent amino acids, whose stereochemical structure is often complementary to that of the drug and which, sometimes after undergoing conformational change, is able to interact with it, usually in its preferred conformation, in order to form a complex held together by various binding forces. As a result of this drug-receptor complexation a stimulus is generated which causes a biological action or effect.

Methylxanthines

- The methylxanthines most widely used in medicine are caffeine, theophylline, and theobromine. In small doses they are taken especially as coffee, tea, cocoa, and chocolate drinks, in order to enhance

mental alertness and wakefulness, to lessen fatigue and to produce diuresis. Excessive amounts, however, cause insomnia and restlessness in some people. Large doses of caffeine and its salts were once used to stimulate the respiratory centre, but now they are not recommended as respiratory stimulants.

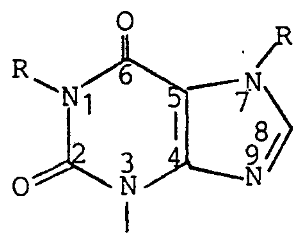

R = R′ = $CH_3$ caffeine
R = H, R′ = $CH_3$ theophylline
R = $CH_3$, R′ = H theobromine

Structure of Methylxanthines

Of the three xanthines shown above, caffeine is the most potent cerebral stimulant; theophyllaine (Theocron) follows, whereas theobromine is almost devoid of stimulant properties. Theophylline produces more diuresis than theobromine and the latter more than caffeine.

Some derivatives of caffeine or theophylline with psychomotor stimulant activity are available; aminophylline (theophylline ethylenediamine), cafedrine (a hybrid of theophylline with ephedrine), dimethazan [7-(2-dimethylaminoethyl) theophylline], fencamine (a hybrid of caffeine with methamphetamine), fenethylline (a hybrid of theophylline with amphetamine), and theodrenaline (a hybrid of theophylline with adrenalin (epinephrine)).

Theophylline and caffeine are used in the treatment of primary apnea of prematurity, as adjuncts to nondrug measures. Maintenance dosage is individualised, because the infant's ability to clear these drugs presents a wide variation. The most common effect is tachycardia, but vomiting and gastrointestinal bleeding may occur. Overdosage can produce convulsions. Theophylline may be given orally by nasogastric tube or syrup, rectally as an enema, or intravenously as aminophylline.

## Bronchodilators

- Xanthine derivatives, because they relax smooth muscle contraction of bronchioles, are useful as adjuncts in the symptomatic treatment of asthma, bronchitis, emphysaema and other obstructive pulmonary disorders. Xanthine derivatives useful as bronchodilators include: acefylline piperazine, acitemate, aminophylline (Phyllocontin, Somphyllin), bamifylline, diniprofylline, dyphylline (diprophylline, Dilor, Lufyllin), doxofylline, enprofylline, etamiphyllin, etofylline, flufylline, fluprophylline, guaithylline (guaifylline, Eclabron), metescufylline, oxtriphylline (Choledyl), pentifylline, proxyphylline, pyridofylline, and theophylline (Accurbron, Bronkodyl, Elixicon, Elixophyllin, Slo-phyllin, Theobid, Theolair, Theon, Theophyl, Theovent, Uniphyl).

The following Examples are given for the purpose of illustration only and are not to be considered as limiting to the invention disclosed herein.

## Examples 1 to 6

Non-cross reactive antibodies to theophylline and the drugs disclosed earlier herein are made according to the methods disclosed in US-A-4262089 incorporated herein by this reference.

In these examples, all percentages not otherwise indicated are by weight, except for mixtures of liquids which are by volume. All temperatures not otherwise indicated are centigrade. Abbreviations include DMF for N,N-dimethylformamide; TLC, thin layer chromatography; ECDI, 1-ethyl-3-(dimethylaminopropyl) carbodiimide hydrochloride; THF, tetrahydrofuran.

Example 1

Preparation of 1-methyl-7-pivaloyloxymethyl xanthine

To a mixture of 1-methylxanthine (844mg, 5.08 mmoles) and sodium carbonate (538mg, 5.08 mmoles) is 20ml of dry DMF under nitrogen was slowly added over a period of 1 hour a solution of chloromethylpivalate (828 µl, 5.59 mmole) in 6ml DMF at room temperature and the reaction mixture stirred overnight. The mixture was filtered, the filtrate evaporated to dryness and the residue triturated with 50mb of 10 vol % EtOH/$CHCl_3$. The solids obtained were starting material and the organic phase was chromatographed (10 vol % EtOH/$CHCl_3$) on silica gel plates. Two products were observed by eluting with 125ml of 10 vol % EtOH/$CHCl_3$ and the band $R_f$, 0.48 obtained as 365mg of the desired product. The other band proved to be the 3,7-disubstituted material.

Example 2

1-methyl-3-(carboethoxypropyl)-7-(pivaloyloxymethyl)xanthine

A mixture of 1-methyl-7-(pivaloyloxymethyl)xanthine (350mg, 1.25 mmes) sodium carbonate (265mg, 2.50 mmoles) and ethyl 4-iodobutyrate (384 µl, 2.50 mmole) in 5.8ml dry DMF was stirred under nitrogen at room temperature for 18 hours. Water was added and the reaction mixture extracted with chloroform. After drying, the extracts were stripped to leave a yellow oil which goes to chromatographed on four thick silica gel plates with 10% EtOH/$CHCl_3$. The band was scraped off and eluted with 100ml of 25% EtOH/$CHCl_3$ to yield 609mg of the desired product as an oil.

Example 3

Preparation of 1-methyl-3-(3′-carboxypropyl)xanthine

An oily suspension of 1-methyl-3(carboethoxypropyl)-7-(pivaloyloxymethyl)xanthine (508mg) in 2N NaOH (36.2ml) was heated under nitrogen at 95°-100° for 2 hrs. The oil dissolved and the reaction was shown to be complete by TLC. After cooling the reaction mixture, it was acidified to pH 2-3 with 15ml 12% hydrochloric acid and the solution extracted with 3x5ml chloroform to remove the pivalic acid. After washing the chloroform extracts with 5ml 0.5N HCl, aqueous solutions were combined nd stripped to dryness and the residue dried in vacuo at room temperature. The crude product was dissolved in about 22ml hot water, decolourised and concentrated to 15ml, at which time 159mg of a crystalline product was obtained upon cooling. mp 220°-221°.

Example 4

Conjugation of 1-methyl-3-(3′-carboxypropyl)xanthine with bovine gamma globulin

To a clear solution of 1-methyl-3-(3-carboxypropyl)xanthine (45mg, 0.178 mmole) in 1.5ml DMF was added N-hydroxysuccinimide (20.5mg, 178 mmole) and EDCI (39.1mg, 0.20 mmole) at 0° under nitrogen. After stirring the solution at 5° for 18 hours, the reaction mixture was added to a solution of bovine gamma globulin (550mg) in a mixture of 27ml carbonate buffer (ph 9, 0.05M) and DMF at 0° and the mixture

maintained at this temperature for a period of 1.5 hours, while maintaining the pH at 8.5-9.0 using 1N NaOH. After stirring the mixture overnight at 5°, the product was dialysed against 10x4 l water and 3x4 l ammonium hydroxide. Lyophilisation of the conjugate yielded 470mg of protein. Employing a spectrophotometric technique, the hapten number was determined to be 15.

Example 5

Preparation of 3-(2′-cyanoethyl)-1-methylxanthine

Into a reaction flask was introduced 7-pivaloyloxymethyl-1-methylxanthine (100mg) dissolved in 1.42ml DMF, 44.17mg sodium carbonate and 47 µl acrylonitrile. After heating at 100° for 16 hours, the mixture was poured into water and the aqueous mixture extracted with chloroform. The chloroform extracts were dried and evaporated and the oily residue chromatographed twice on silica gel plates, and the band eluted with 75ml of 25% ethanol/chloroform (v/v) to give 109mg of an oily product which solidified on standing. mp 118-120.

The above nitrile (109mg) was suspended in 0.35ml 1N sodium hydroxide and 0.35ml of THF added. The mixture was stirred at room temperature for 3.5 hours, followed by the addition of 0.2ml of 1N sodium hydroxide and stirring continued for an additional 0.5 hour. The mixture was then poured into about 2ml water, the aqueous solution acidified and extracted exhaustively with chloroform. The chloroform extracts were washed and 8% sodium bicarbonate, followed by drying and evaporation to dryness. The residue was chromatographed on two silica gel plates (10% $EtOH/HCCl_3$) and eluted with 125ml 25% $EtOH/HCCl_3$. Evaporation to dryness yield 50mg of the desired product.

The subject nitrile can be readily modified to the imidoester and used for conjugation to amino groups present in poly(amino acids) to provide amidine linkages.

Example 6

Conjugation of 3-carboxypropyl-1-methylxanthine to glucose-6-phosphate dehydrogenase

A lyophilised powder of G6PDH was dissolved in 0.055M tris-HCl, pH 8.1, to a protein concentration of about 2-3mg/ml. The mixture was allowed to stand overnight at 4°.

Into a reaction flask was introduced 3-carboxypropyl-1-methylxanthine (36mg, 0.14 mmoles), 16.4mg of N-hydroxy succinimide, 31.4mg of ECDI and 400 µl of DMF and the mixture stirred overnight at 4°.

To 5ml of the above G6PDH solution is added 50mg glucose-6-phosphate disodium salt, 100mg of NADH and 1.5ml of carbitol and the pH adjusted to about 8.5-9 with 2N sodium hydroxide. Almost the entire 400 µl of the ester prepared above is added to the stirring enzyme solution in 10 µl increments over a 2 hour period while maintaining the solution at 4°. During the reaction the pH drops to 7.5-8. The resulting conjugate is then dialysed at 4° against 0.055M tris HCl, pH 8.1, containing as preservatives 0.5% sodium azide and 0.005% thimerosal.

Antitheophylline antibodies are prepared from the conjugates in the normal way.

Examples 7 to 10

Low molecular weight ligands of the non-cross reactive antibodies produced in Example I are made according to the methods shown in PCT Application WO-A-8606487 incorporated herein by this reference.

Example 7

Synthesis of a plurality of catamers

Catamers are synthesised on a solid support. In this embodiment, the plurality of catamers will all have

the general formula:

Y-$D_2$-$D_1$-Lk- (solid support),

where "Lk" represents a linker molecule which provides a suitable end group for condensing the monomers to the solid support. "$D_1$" and "$D_2$", represent designated positions occupied by monomers which are selected from known sets of monomers; but which are altered systematically between catamers. It should be noted that the set of monomers used for the $D_1$ designated position need not be the same set of monomers used for the $D_2$ designated position. "Y" in the general formula is an end group of the catamer and may be, but is not restricted to, for example a hydrogen atom or an acetyl group. "Y" may also be another molecule which is coupled to the catamer to preserve particular characteristics of the molecular environment of a peptide bond at the amino terminal designated position.

If i is the number of members in the set of monomers to be coupled in the $D_1$ position and j is the number of members in the set of monomers to be coupled in the $D_2$ position then, a total of i. j different catamers will be synthesised.

In the present embodiment, the support rods are prepared so that the monomers can be coupled to them by coupling an appropriate linker molecule.

For the coupling at the $D_1$ position, each rod will be treated with a reaction mixture which contains only a single monomer such as a protected amino acid or the like. In this position each of the i monomers are coupled to j rods. For the coupling at the $D_2$ position each rod is treated with a reaction mixture which contains a single monomer such as a protected amino acid or the like. Each of the j rods which has a particular monomer in the $D_1$ position will have a different monomer coupled at the $D_2$ position. In this way every combination of the members of the set(s) of monomers will be found in the i. j rods.

The desired end group, "Y", is then coupled using the appropriate chemistry.

After synthesis of the plurality of catamers any side-chain protective groups are removed from the catamers using the appropriate techniques and the rod-coupled catamers are washed.

It has been found to be a preferred embodiment of the invention to synthesise more than one set of plurality of catamers to aid in the analysis of data. Thus, as well as synthesising catamers with the general formula

Y-$D_2$-$D_1$-Lk-(solid support)

as described above, additional sets of catamers may be prepared with the general formula

Y-$D_2$-Sp-$D_1$-Lk-(solid support)

where "Sp" is a spacer molecule which may restrict the relative orientation of the monomers at the designated positions to a particular geometrical configuration(s). The spacer molecule may also be deliberately chosen to allow a greater flexibility to the relative geometric configuration between monomers in the designated positions, $D_1$ and $D_2$. It should be noted that members of the set of spacer molecules may be made up from the condensation of more than one monomer. Examples of spacer molecules include, but are not restricted to glycine (approximately linear extension), beta-alanine (increased flexibility), proline (forced bend), glycyl-proline (extended bend, otherwise known as reverse bend in protein structure terminology) and o-aminobenzoic acid (a planar bend).

By analysing the results from these sets of catamers the preferred spatial relationships between monomers in the mimotopes can be deduced as will be shown in the appropriate examples given below.

## Example 8

### Testing of the plurality of catamers

The plurality of catamers prepared in Example 7 above are then contacted with the particular antibody of interest. The reaction between antibody and each catamer can then be detected by any of the usual methods, for example, radioimmunoassay (RIA). However, the preferred method of detection is to use the well known enzyme-linked immunosorbent assay (ELISA).

At the end of each assay antibodies can be removed from the catamers by, for example, washing with a solution of 8M urea, 0.1% 2-mercaptoethanol and 0.1% sodium dodecylsulphate followed by several washes in phosphate buffered saline. In this way the plurality of catamers may be used for testing with many other antibodies.

## Example 9

Analyses of the data

In the testing of a set of catamers with antibody it has been found that certain catamers will show detectable binding with the antibody. These reacting catamers identify useful combinations of the members of the set(s) of monomers. These combinations of monomers are short mimotopes which, when extended, may bind to the antibody with a greater affinity or alters specificity. Analysis of the data is greatly facilitated by including a number of control peptides in the synthesis which aid the determination of significant responses. Further analysis of the data can be carried out in a number of ways. These include:

1. permutating each of these reacting combinations of monomers to create a list of catamers which will include mimotopes which bind to the paratope; each catamer in this list can be regarded as a possible mimotope;

2. selecting candidates from the list of reacting combinations of monomers and further synthesising sets of catamers in which the known reacting combination of monomers is held constant and further monomers are added systematically at either end; thus in the example above, a suitable set of such catamers would include catamers with the formulae

$Y-D_3-A_2-A_1-Lk$-(solid support)

and

$Y-A-_2-A_1-D_3-LK$-(solid support)

where $A_1$ and $A_2$ constitute the reacting combination of monomers from the previous results and $D_3$ is the new designated position where each of the members of the set of monomers is systematically varied; and

3. combining the results from different sets of a plurality of catamers and analysing the results to deduce a single sequence of monomers, or a small number of such sequences which would bind to the antibody of interest. Analysis of this data is possible as the reacting combinations of monomers when they are adjacent to each other are now known, as well as the reacting combinations of monomers when they have particular geometrical configurations between them. In this way, these data can then be interpreted to predict the structure of the mimotope when bound to the antibody; this approach will be of greatest benefit when the antibody used to test the plurality of catamers is a monoclonal antibody.

The procedure given in 2 above can be repeated until no further enhancement of binding is achieved by additional extending of the mimotope. Ideally, the sequence of the mimotope should be checked by regularly synthesising and testing replacement nets of the mimotope to obtain the optimally-binding mimotope for further extension as describe din AU-A-8425428.

Example 10

Synthesis of selected catamers

The selected catamers can be synthesised using similar methods to those used in Example 7. After the selected catamers have been synthesised they are reacted with the antibody of interest. It is then simple to select the catamer which binds most strongly with the antibody.

The binding of the mimotope with the antibody can be further enhanced by synthesising a further plurality of catamers based on the sequence of the most strongly binding mimotope. This plurality of catamers consists of adding spacer molecules (-Sp-) systematically at all positions of the mimotope; and where feasible systematically replacing each monomer of the catamer with its optical isomer. Testing of this set of catamers with the antibody will give invaluable information about the relative orientation of the monomers and their stereochemistry as required for binding with the antibody.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

## Claims

1. A method of producing a low molecular weight ligand which is capable of binding to a naturally occurring receptor comprising producing non-crossreactive antibodies to a haptenic substance and producing the ligand as a low molecular weight structural mimic of the antibody.

2. A method of producing a low molecular weight ligand capable of tightly binding to a naturally occurring receptor comprising the sequential steps of (1) attaching a haptenic substance to a macromolecular carrier at a point on the haptenic substance having a common structural feature to related haptenic substances, (2) introducing the resulting carrier-haptenic combination into a live animal to produce non-crossreactive antibodies to the haptenic substance, (3) harvesting the antibodies and (4) producing the ligand as a low molecular weight structural mimic of the antibody.

3. A method of producing a low molecular weight ligand capable of bronchodilation comprising the sequential steps of (1) attaching theophylline to a macromolecular carrier at a point on the theophylline molecule having the common structural features of methyl xanthins, (2) introducing the resulting carrier-theophylline combination into a live animal to produce non-crossreactive antibodies to the theophylline, (3) harvesting the antibodies, and (4) producing the ligand as a low molecular weight structural mimic of the antibody.

4. A method as claimed in claim 1, 2 or 3, wherein the ligand is made from L-amino acids and/or D-amino acids, preferably L-amino acids.

5. A method as claimed in any one of claims 1 to 4, wherein the ligand is a drug, a hormone analogue or wherein the ligand has the pharmacological activity of a naturally occurring polypeptide, such as a hormone, an interleukin or an interferon.

6. A method as claimed in any one of claims 1 to 5, wherein the non-crossreactive antibody is monoclonal.

7. A method as claimed in claim 2, wherein the haptenic substance is a drug.

8. A method as claimed in claim 5, wherein the drug is theophylline or porcine somatotropin.

9. A method as claimed in claim 3, wherein the macromolecular carrier is attached to the theophylline through the N-3 position.

10. The use of non-crossreactive antibodies in the production of a compound capable of binding to a naturally occurring receptor.

European Patent Office

# EUROPEAN SEARCH REPORT

Application Number

EP 90 30 3499

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.5) |
| Y | BIOLOGICAL ABSTRACTS, vol. 87, no. 7, abstract no. 70311, Biological Abstracts, Inc., Philadelphia, PA, US; R. TAUB et al.: "A monoclonal antibody against the platelet Fibrinogen receptor contains a sequence that mimics a receptor recognition domain in fibrinogen", & J. BIOL. CHEM. 264(1): 259-265. 1989<br>* Abstract *<br>--- | 1 | C 12 P 21/08<br>C 07 K 3/00 |
| Y | BIOLOGICAL ABSTRACTS, vol. 82, no. 7, 1986, abstract no. 62206, Biological Abstracts, Inc., Philadelphia, PA, US; L.H.K. DEFIZE et al.: "Dissociation of cellular responses to epidermal growth factor using anti-receptor monoclonal antibodies", & EMBO (EUR. MOL. BIOL. ORGAN.) J 5(6): 1187-1192, 1986<br>* Abstract *<br>----- | 1 | |
| | | | TECHNICAL FIELDS SEARCHED (Int. Cl.5) |
| | | | C 12 P |

The present search report has been drawn up for all claims

| Place of search | Date of completion of the search | Examiner |
|---|---|---|
| THE HAGUE | 29-06-1990 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document